# EUROPÄISCHE PATENTSCHRIFT

(11) **EP 1 472 250 B1**
(45) Veröffentlichungstag und Bekanntmachung des Hinweises auf die Patenterteilung: **26.11.2014**
(21) Anmeldenummer: 03704441.9
(22) Anmeldetag: 21.01.2003
(51) Int. Cl.: C07D 451/10, A61K 31/46, A61P 1/06, A61P 11/06, A61P 13/06, A61P 9/06

(54) **NEUE ANTICHOLINERGIKA, VERFAHREN ZU DEREN HERSTELLUNG SOWIE DEREN VERWENDUNG ALS ARZNEIMITTEL**
ANTICHOLINERGIC AGENTS, METHOD FOR PRODUCING THE SAME AND USE THEREOF AS MEDICAMENTS
SUBSTANCES ANTICHOLINERGIQUES, PROCÉDÉ DE PRÉPARATION ASSOCIÉ ET UTILISATION COMME MÉDICAMENT

(30) Priorität: 31.01.2002 DE 10203749
(43) Veröffentlichungstag der Anmeldung: 03.11.2004
(73) Patentinhaber: Boehringer Ingelheim Pharma GmbH & Co. KG, 55216 Ingelheim am Rhein (DE)
(72) Erfinder: SPECK, Georg, 55218 INGELHEIM AM RHEIN (DE); EICKMEIER, Christian, 88441 MITTELBIBERACH (DE); PESTEL, Sabine, 88400 BIBERACH (DE); GERMEYER, Sabine, 88400 BIBERACH (DE); PIEPER, Michael, P., 88400 BIBERACH (DE); BREITFELDER, Steffen, 88433 AssMANNSHARDT (DE); GRAUERT, Matthias, 88400 BIBERACH (DE)
(86) Internationale Anmeldenummer: PCT/EP2003/000533
(87) Internationale Veröffentlichungsnummer: WO 2003/064418

(56) Entgegenhaltungen:
- EP-A- 0 418 716
- WO-A-92/16528
- DISSE B ET AL: "BA 679 BR, A NOVEL LONG-ACTING ANTICHOLINERGIC BRONCHODILATOR" LIFE SCIENCES, PERGAMON PRESS, OXFORD, GB, Bd. 52, Nr. 5/6, 1993, Seiten 537-544, XP008002589 ISSN: 0024-3205
- DATABASE CA [Online] CHEMICAL ABSTRACTS SERVICE, COLUMBUS, OHIO, US; DISSE, BERND ET AL: "Tiotropium (Spiriva): mechanistical considerations and clinical profile in obstructive lung disease" retrieved from STN Database accession no. 130:261837 CA XP002235757 & LIFE SCIENCES (1999), 64(6/7), 457-464 , 1999,

## Beschreibung

Die vorliegende Erfindung betrifft neue Anticholinergika der allgemeinen Formel **1** 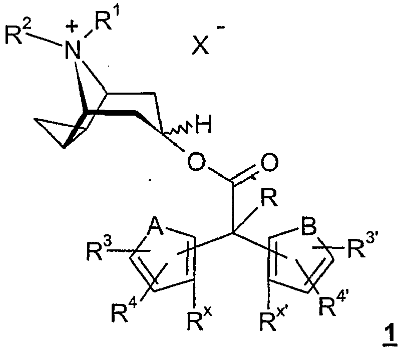 worin X⁻ und die Reste A, B, R, R¹, R², R³, R^{3'}, R⁴, R^{4'}, R^{X} und R^{X'}, die in den Ansprüchen und in der Beschreibung genannten Bedeutungen haben können, und die Verbindungen der allgemeine Formel 1 zur Verwendung als Arzneimittel. EP 418 716 A1 und WO 92/16528 beschreiben Scopin-Verbindungen als Anticholinergika.

### Beschreibung der Erfindung

Die vorliegende Erfindung betrifft Verbindungen der allgemeinen Formel **1** 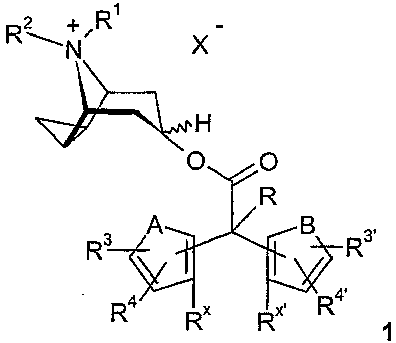 worin
- X⁻: ein einfach negativ geladenes Anion, vorzugsweise ein Anion ausgewählt aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p-Toluolsulfonat;
- A und B: gleich oder verschieden, bevorzugt gleich, -O-, -S-, -NH-, -CH₂-, -CH=CH-, oder -N(C₁-C₄-Alkyl)-;
- R: Wasserstoff, Hydroxy, -C₁-C₄-Alkyl, -C₁-C₄-Alkyloxy, -C₁-C₄ -Alkylen-Halogen, -O-C₁-C₄-Alkylen-Halogen, -C₁-C₄-Alkylen-OH, -CF₃, CHF₂, -C₁-C₄-Alkylen-C₁-C₄-alkyloxy, -O-COC₁-C₄-Alkyl, -O-COC₁-C₄-Alkylen-Halogen, -C₁-C₄-Alkylen-C₃-C₆-Cycloalkyl, -O-COCF₃ oder Halogen;
- R¹ und R²: gleich oder verschieden, -C₁-C₅-Alkyl, welches gegebenenfalls durch -C₃-C₆-Cycloalkyl, Hydroxy oder Halogen substituiert sein kann, oder R¹ und R² gemeinsam eine -C₃-C₅-Alkylen-Brücke;
- R³, R⁴, R^{3'} und R^{4'},: gleich oder verschieden, Wasserstoff, C₁-C₄-Alkyl, C₁-C₄-Alkyloxy, Hydroxy, -CF₃, -CHF₂, CN, NO₂ oder Halogen;
- R^{X} und R^{X'}: gleich oder verschieden Wasserstoff, C₁-C₄-Alkyl, C₁-C₄-Alkyloxy, Hydroxy, -CF₃, -CHF₂, CN, NO₂ oder Halogen oder
R^{X} und R^{X'} gemeinsam ein Einfachbindung oder eine verbrückende Gruppe ausgewählt aus den Brücken -O-, -S-, -NH-, -CH₂-, -CH₂-CH₂-, -N(C₁-C₄-Alkyl)-, -CH(C₁-C₄-Alkyl)- und -C(C₁-C₄-Alkyl)₂-, bedeuten.

Bevorzugt sind Verbindungen der allgemeinen Formel **1**, worin
- X⁻: ein einfach negativ geladenes Anion ausgewählt aus der Gruppe Chlorid, Bromid, 4-Toluolsulfonat und Methansulfonat, bevorzugt Bromid;
- A und B: gleich oder verschieden, bevorzugt gleich, -O-, -S-, -NH- oder -CH=CH-;
- R: Wasserstoff, Hydroxy, -C₁-C₄-Alkyl, -C₁-C₄-Alkyloxy, -CF₃, -CHF₂, Fluor, Chlor oder Brom;
- R¹ und R²: gleich oder verschieden, C₁-C₄-Alkyl, welches gegebenenfalls durch Hydroxy, Fluor, Chlor oder Brom substituiert sein kann,
oder
R¹ und R² gemeinsam eine -C₃-C₄-Alkylen-Brücke;
- R³, R⁴, R^{3'} und R^{4'},: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom;
- R^{x} und R^{x'}: gleich oder verschieden Wasserstoff, C₁-C₄-Alkyl, C₁-C₄-Alkyloxy, Hydroxy, -CF₃, -CHF₂, CN, NO₂, Fluor, Chlor oder Brom
oder
R^{X} und R^{X'} gemeinsam ein Einfachbindung oder eine verbrückende Gruppe ausgewählt aus den Brücken -O-, -S-, -NH- und -CH₂-bedeuten.

Besonders bevorzugt sind Verbindungen der allgemeinen Formel **1**, worin
- X⁻: ein einfach negativ geladenes Anion ausgewählt aus der Gruppe Chlorid, Bromid und Methansulfonat, bevorzugt Bromid;
- A und B: gleich oder verschieden, bevorzugt gleich, -S- oder -CH=CH-;
- R: Wasserstoff, Hydroxy, Methyl, Ethyl, Methyloxy, Ethyloxy, -CF₃, oder Fluor;
- R¹ und R²: gleich oder verschieden, Methyl, Ethyl, -CH₂F oder -CH₂-CH₂F, bevorzugt Methyl oder Ethyl;
- R³, R⁴, R^{3'} und R^{4'},: gleich oder verschieden, Wasserstoff, Methyl, Methyloxy, -CF₃ oder Fluor;
- R^{X} und R^{X'}: gleich oder verschieden Wasserstoff, Methyl, Methyloxy, -CF₃ oder Fluor
oder
R^{X} und R^{X'} gemeinsam ein Einfachbindung oder die verbrückende Gruppe -O- bedeuten.

Erfindungsgemäß von besonderer Bedeutung sind Verbindungen der allgemeinen Formel **1**, worin
- X⁻: ein einfach negativ geladenes Anion ausgewählt aus der Gruppe Chlorid, Bromid und Methansulfonat, bevorzugt Bromid;
- A und B: gleich oder verschieden, bevorzugt gleich, -S- oder -CH=CH-;
- R: Wasserstoff, Hydroxy oder Methyl;
- R¹ und R²: gleich oder verschieden, Methyl oder Ethyl;
- R³, R⁴, R^{3'} und R^{4'},: gleich oder verschieden, Wasserstoff, -CF₃ oder Fluor, bevorzugt Wasserstoff;
- R^{X} und R^{X'}: gleich oder verschieden Wasserstoff, -CF₃ oder Fluor, bevorzugt Wasserstoff oder
R^{X} und R^{X'} gemeinsam ein Einfachbindung oder die verbrückende Gruppe -O- bedeuten.

Erfindungsgemäß bevorzugt sind ferner Verbindungen der allgemeinen Formel **1**, worin
- X⁻: Bromid;
- A und B: -CH=CH-;
- R: Wasserstoff, Hydroxy oder Methyl;
- R¹ und R²: Methyl;
- R³, R⁴, R^{3'} und R^{4'},: gleich oder verschieden, Wasserstoff oder Fluor, bevorzugt Wasserstoff;
- R^{X} und R^{X'}: gleich oder verschieden Wasserstoff oder Fluor, bevorzugt Wasserstoff
oder
R^{X} und R^{X'} gemeinsam ein Einfachbindung oder die verbrückende Gruppe -O- bedeuten.

Gegenstand der Erfindung sind die jeweiligen Verbindungen der Formel 1 gegebenenfalls in Form der einzelnen optischen Isomeren, Mischungen der einzelnen Enantiomeren oder Racemate sowie gegebenenfalls in Form ihrer pharmakologisch verträglichen Säureadditionssalze.

In den Verbindungen der allgemeinen Formel 1 können die Reste R³, R⁴, R^{3'} und R^{4'}, sofern sie nicht Wasserstoff bedeuten, jeweils ortho, meta oder para bezüglich der Verknüpfung zur "-C- R"-Gruppe angeordnet sein. Sofern keiner der Reste R³, R⁴, R^{3'} und R^{4'} Wasserstoff bedeutet, sind R³ und R^{3'} bevorzugt in para-Position und R⁴ und R^{4'} bevorzugt in ortho- oder meta-Position, besonders bevorzugt in meta-Position verknüpft. Sofern einer der Reste R³ und R⁴ und einer der Reste R^{3'} und R^{4'} Wasserstoff bedeutet, ist der jeweils andere Rest bevorzugt in meta- oder para-Position, besonders bevorzugt in para-Position verknüpft. Sofern keiner der Reste R³, R⁴, R^{3'} und R^{4'} Wasserstoff bedeutet sind erfindungsgemäß die Verbindungen der allgemeinen Formel **1** besonders bevorzugt, in denen die Reste R³, R⁴, R^{3'} und R^{4'} dieselbe Bedeutung aufweisen.

Von besonderer Bedeutung sind erfindungsgemäß die Verbindungen der allgemeinen Formel 1, in denen die beiden Ringe, die A und B, enthalten, so angeordnet sind, daß A und B bezogen auf die Verknüpfung zum "C-R" Kohlenstoff jeweils ortho-konfiguriert sind. Diese bevorzugte Konfoguration kommt insbesondere dann zum Tragen, wenn A und B nicht -CH=CH- bedeuten. Diese Verbindungen entsprechen der allgemeinen Formel ***ortho-1***.

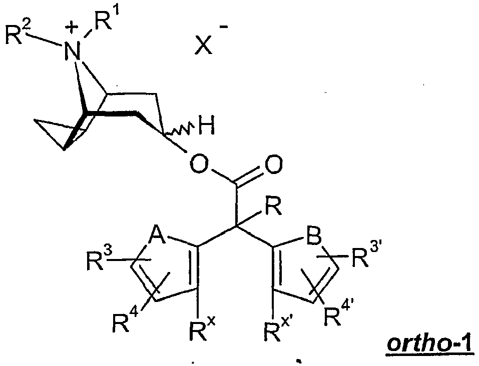

Von besonderer Bedeutung sind erfindungsgemäß die Verbindungen der allgemeinen Formel 1, in denen A -CH=CH- und B -CH=CH- bedeuten. Diese Verbindungen entsprechen der allgemeinen Formel 1'.

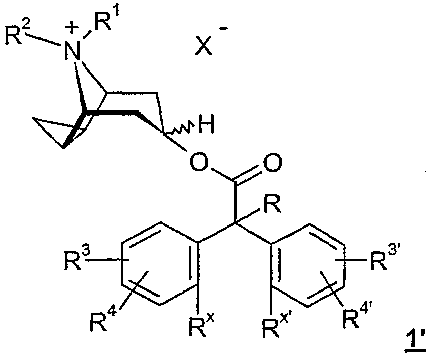

Von besonderer Bedeutung sind erfindungsgemäß ferner diejenigen Verbindungen der allgemeinen Formel **1**, in denen der Ester-substituent am Stickstoff-bicylus α-konfiguriert ist. Diese Verbindungen entsprechen der allgemeinen Formel **1-α**

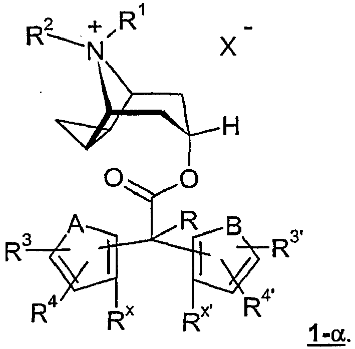

Die erfindungsgemäß bevorzugten Verbindungen der allgemeinen Formel 1, in denen die beiden Ringe, die A und B, enthalten, so angeordnet sind, daß A und B bezogen auf die Verknüpfung zum "C-R" Kohlenstoff jeweils ortho-konfiguriert sind und in denen darüberhinaus der Ester-substituent am Stickstoff-bicylus α-konfiguriert ist entsprechen der allgemeinen Formel ***ortho*-1-α**

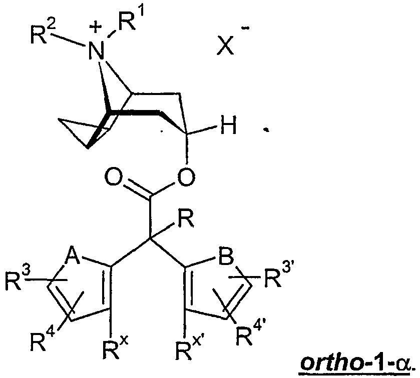

Die erfindungsgemäß besonders bevorzugten Verbindungen der allgemeinen Formel **1**, in denen A -CH=CH- und B -CH=CH- bedeuten und in denen der Ester-substituent am Stickstoff-bicylus α-konfiguriert ist entsprechen der allgemeinen Formel **1'-α**.

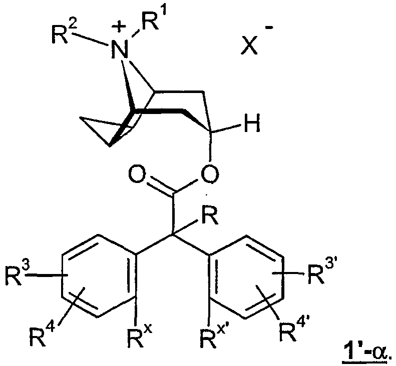

Die nachfolgenden Verbindungen sind erfindungsgemäß von besonderer Bedeutung:
- Benzilsäurecyclopropyltropinester-Methobromid;
- 2,2-Diphenylpropionsäurecyclopropyltropinester -Methobromid;
- 9-Hydroxy-xanthen-9-carbonsäurecyclopropyltropinesterMethobromid;
- 9-Methyl-fluoren-9-carbonsäurecyclopropyltropinester-Methobromid;
- 9-Methyl-xanthen-9-carbonsäurecyclopropyltropinester-Methobromid;
- 9-Hydroxy-fluoren-9-carbonsäurecyclopropyltropinester -Methobromid ;
- 4,4'-Difluorbenzilsäuremethylestercyclopropyltropinester-Methobromid.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Cycloalkylgruppen werden, soweit nicht anders angegeben, alicyclische Gruppen mit 3 bis 6 Kohelnstoffatomen verstanden. Hierbei handelt es sich um die Gruppen Cyclopropyl, Cyclobutyl, Cyclopentyl und Cyclohexyl. Erfindungsgemäß von besonderer Bedeutung ist im Rahmen der vorliegenden Erfindung Cyclopropyl.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, Propylen oder Butylen.

Als Alkylen-Halogen-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt zweifach durch ein Halogen substituiert sind. Dementsprechend werden als Alkylen-OH-Gruppen, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch ein Hydroxy substituiert sind.

Als Alkyloxygruppen werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Alkylen-alkyloxy-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch eine Alkyloxygruppe substituiert sind.

Als -O-CO-Alkylgruppen werden, soweit nicht anders angegeben, verzweigte und unverzweigte Alkylgruppen mit 1 bis 4 Kohlenstoffatomen bezeichnet, die über eine Estergruppe verknüpft sind. Dabei sind die Alkylgruppen direkt an den Carbonylkohlenstoff der Estergruppe gebunden. In analoger Art und Weise ist die Bezeichnung -O-CO-Alkyl-Halogen-gruppe zu verstehen. Die Gruppe -O-CO-CF₃ steht für Trifluoracetat.

Halogen steht im Rahmen der vorliegenden Erfindung für Fluor, Chlor, Brom oder Jod. Sofern nicht gegenteilig angegeben, gelten Fluor und Brom als bevorzugte Halogene. Die Gruppe CO bezeichnet eine Carbonylgruppe.

Die Herstellung der erfindungsgemäßen Verbindungen kann, wie nachstehend erläutert, zum Teil in Analogie zu im Stand der Technik bereits bekannten Vorgehensweisen erfolgen (Schema 1). Die Carbonsäurederivate der Formel **3** sind im Stand der Technik bekannt oder können nach im Stand der Technik bekannten Syntheseverfahren erhalten werden. Sind lediglich geeignet substituierte Carbonsäuren im Stand der Technik bekannt, können die Verbindungen der Formel **3** auch direkt aus diesen durch Säure- oder Basen-katalysierte Veresterung mit den entsprechenden Alkoholen oder durch Halogenierung mit den entsprechenden Halogenierungsreagentien erhalten werden.

Ausgehend von den Verbindungen der Formel **2** gelingt der Zugang zu den Estern der allgemeinen Formel **4** durch Umsetzung mit den Carbonsäurederivaten der Formel **3**, in denen R' beispielsweise für Chlor oder einen C₁-C₄-Alkyloxyrest steht. Im Falle von R' gleich C₁-C₄-Alkyloxy kann diese Umsetzung beispielsweise in einer Natriumschmelze bei erhöhter Temperatur, bevorzugt bei ca. 50-150°C, besonders bevorzugt bei etwa 90-100°C bei niedrigem Druck, bevorzugt bei unter 500mbar, besonders bevorzugt bei unter 75mbar durchgeführt werden. Alternativ dazu können statt der Derivate **3**, in denen R' C₁-C₄-Alkyloxy bedeutet auch die entsprechenden Säurechloride (R gleich Cl) eingesetzt werden.

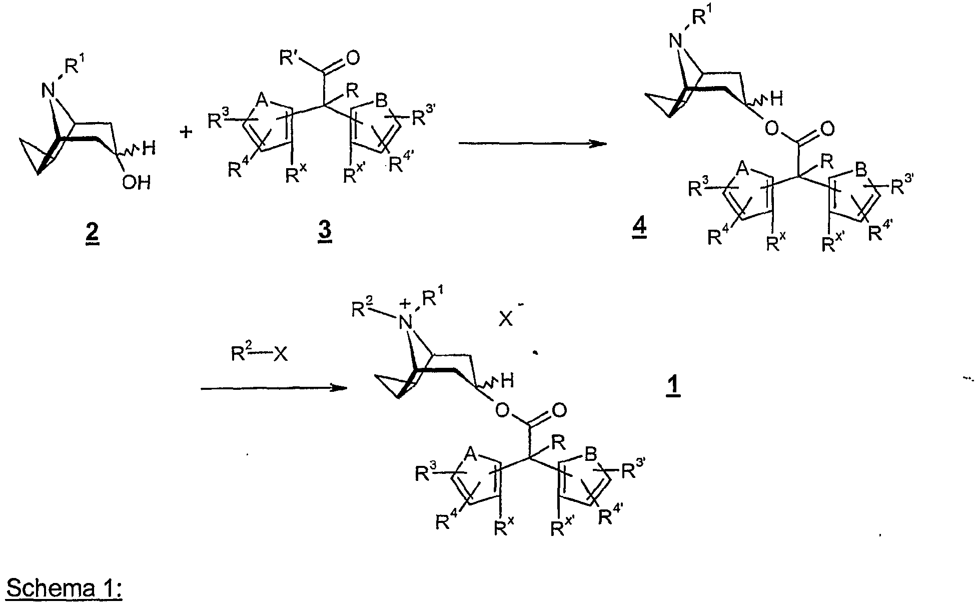

Die so erhaltenen Verbindungen der Formel **4** lassen sich durch Umsetzung mit den Verbindungen R²-X, in denen R² und X die vorstehend genannten Bedeutungen haben können, in die Zielverbindungen der Formel **1** überführen. Auch die Durchführung dieses Syntheseschritts kann in Analogie zu den in der WO 92/16528 offenbarten Synthesebeispielen erfolgen. In dem Fall in dem R¹ und R² gemeinsam eine Alkylenbrücke bilden, ist, wie für den Fachmann ersichtlich, die Zugabe des Reagenzes R²-X nicht erforderlich. In diesem Fall weisen die Verbindungen der Formel **4** einen geeignet substituierten Rest R¹ (beispielsweise -C₃-C₅-Alkylen-Halogen) entsprechend der vorstehend genannten Definitionen auf und die Darstellung der Verbindungen der Formel **1** erfolgt durch intramolekulare Quarternierung des Amins.

Alternativ dazu sind die Verbindungen der Formel **4**, in denen R Halogen bedeutet auch auf dem in Schema 2 dargestellten Wege zugänglich.

Hierzu werden die Verbindungen der Formel **4** in denen R für Hydroxy steht unter Verwendung geeigneter Halogenierungsreagentien in die Verbindungen **4**, in denen R Halogen bedeutet, überführt. Die Durchführung der nach Schema 2 durchzuführenden Halogenierungsreaktionen ist im Stand der Technik hinreichend bekannt.

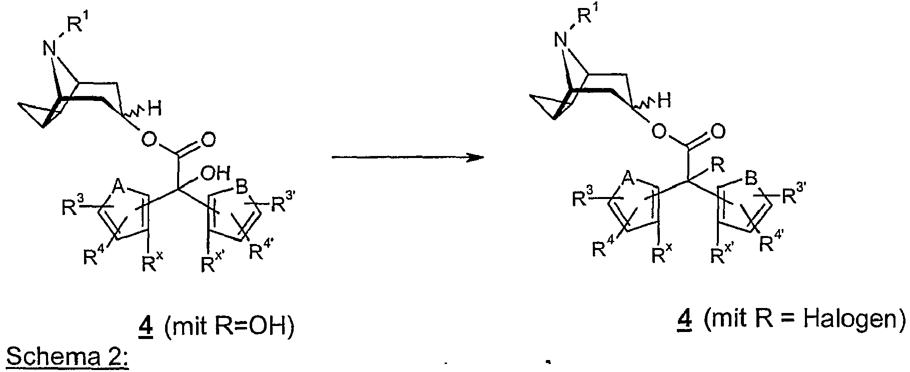

Wie in Schema 1 ersichtlich, kommt den Zwischenprodukten der allgemeinen Formel **4** eine zentrale Bedeutung zu. Dementsprechend zielt ein weiterer Aspekt der vorliegenden Erfindung auf die Intermediate der Formel **4** 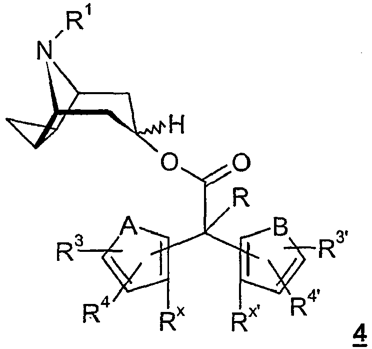 worin die Reste A, B, R, R¹, R³, R^{3'}, R⁴, R^{4'}, R^{X} und R^{X'} die vorstehend genannten Bedeutungen haben können, gegebenenfalls in Form ihrer Säureadditionssalze. Unter Säureadditionssalzen werden dabei Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Wie in den Verbindungen der allgemeinen Formel **1** können auch in den Verbindungen der allgemeinen Formel **4** die Reste R³, R⁴, R^{3'} und R^{4'}, sofern sie nicht Wasserstoff bedeuten, jeweils ortho, meta oder para bezüglich der Verknüpfung zur "-C- R"-Gruppe angeordnet sein. Sofern keiner der Reste R³, R⁴, R^{3'} und R^{4'} Wasserstoff bedeutet, sind R³ und R^{3'} bevorzugt in para-Position und R⁴ und R^{4'} bevorzugt in ortho- oder meta-Position, besonders bevorzugt in meta-Position verknüpft. Sofern einer der Reste R³ und R⁴ und einer der Reste R^{3'} und R^{4'} Wasserstoff bedeutet, ist der jeweils andere Rest bevorzugt in meta- oder para-Position, besonders bevorzugt in para-Position verknüpft. Sofern keiner der Reste R³, R⁴, R^{3'} und R^{4'} Wasserstoff bedeutet sind erfindungsgemäß die Verbindungen der allgemeinen Formel **4** besonders bevorzugt, in denen die Reste R³, R⁴, R^{3'} und R^{4'} dieselbe Bedeutung aufweisen.

Wie aus Schema 1 ersichtlich, dienen als Ausgangsprodukte für die Darstellung der Verbindungen der Formel **1** die Verbindungen der Formel **2**. Diese Verbindungen sind im Stand der Technik noch nicht bekannt. Dementsprechend zielt ein weiterer Aspekt der vorliegenden Erfindung auf Verbindungen der allgemeinen Formel **2** 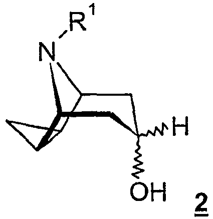 worin
- R¹: Wasserstoff oder -C₁-C₅-Alkyl, welches gegebenenfalls durch -C₃-C₆-Cycloalkyl, Hydroxy oder Halogen substituiert sein kann, bedeutet, gegebenenfalls in Form Ihrer Säureadditionssalze.

Unter Säureadditionssalzen werden dabei Salze ausgewählt aus der Gruppe bestehend aus Hydrochlorid, Hydrobromid, Sulfat, Phosphat, Fumarat und Methansulfonat verstanden. Bevorzugt sind Verbindungen der allgemeinen Formel **2**, worin
- R¹: Wasserstoff oder C₁-C₄-Alkyl, welches gegebenenfalls durch Hydroxy, Fluor, Chlor oder Brom substituiert sein kann, bedeutet, gegebenenfalls in Form Ihrer Säureadditionssalze.

Besonders bevorzugt sind Verbindungen der allgeminen Formel **2**, worin
- R¹: Wasserstoff, Methyl, Ethyl, -CH₂F oder -CH₂-CH₂F, bevorzugt Methyl oder Ethyl, bedeutet, gegebenenfalls in Form Ihrer Säureadditionssalze.

Erfindungsgemäß von besonderer Bedeutung sind Verbindungen der allgemeinen Formel **2**, worin
- R¹: Wasserstoff, Methyl oder Ethyl bedeutet, gegebenenfalls in Form Ihrer Säureadditionssalze.

Erfindungsgemäß bevorzugt sind ferner Verbindungen der allgemeinen Formel **2**, worin
- R¹: Wasserstoff oder Methyl bedeutet, gegebenenfalls in Form Ihrer Säureadditionssalze.

Erfindungsgemäß bevorzugt werden die Verbindungen der Formel **2** in α-konfigurierter Form als Ausgangsmaterialien eingesetzt. Diese α-konfigurierten Verbindungen sind erfindungsgemäß demzufolge von besonderer Bedeutung und entsprechen der allgemeinen Formel **2-α**.

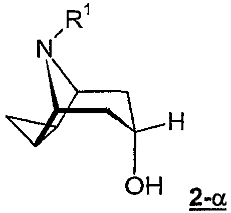

Verbindungen der allgemeinen Formel 2-α, mit R¹ = Methyl werden im folgenden als Cyclopropyltropin bezeichnet. Dabei wird bei der Alkoholgruppe die α-Stellung und bei der Cyclopropylgruppe die *Exo*-Konfiguration vorausgesetzt. (Cyclopropyltropin = *Exo*-Cyclopropyl-α-tropin). Die β-konfigurierte Verbindung wird gegebenenfalls als *Pseudo*-Cyclopropyltropin bezeichnet und das *Endo*-Isomere als *Endo-*Cyclopropyltropin.

Ein weiterer Aspekt der vorliegenden Erfindung betrifft die Verwendung von Verbindungen der allgemeinen Formel **2** zur Herstilung der Verbindungen der allgemeinen Formel **4**. Ferner betrifft die vorliegende Erfindung die Verwendung der Verbindungen der allgemeinen Formel **2** als Ausgangsmaterial zur Herstellung der Verbindungen der allgemeinen Formel **1**.

Die Verbindungen der allgemeinen Formel **2** lassen sich in Analogie zu im Stand der Technik bekannten Vorgehensweisen ausgehend von den entsprechenden Tropenol-Derivaten erhalten. Als geeignete Cyclopropylierungsreagentien kommt hierbei beispielsweise Diazomethan in Betracht.

Die nachstehend beschriebenen Synthesebeispiele dienen der weitergehenden Illustration der vorliegenden Erfindung.

### Darstellung der Verbindung der Formel 2:

### Cyclopropyltropin 2a:

35 ml (0,35 mol) 40%-ige wässrige Kaliumhydroxidlösung wird mit 100 ml Diethylether überschichtet und im Eisbad gekühlt. Hierzu werden 23,64 g (0,101 mol) N-Methyl-N-Nitrosoharnstoff portionsweise zugegeben und anschließend etwa 10 Minuten nachgerührt. Die Etherphase wird abdekantiert und die erhaltene Lösung im folgenden Syntheseschritt eingesetzt.

Zu einer Lösung aus 4,01 g (0,028 mol) Tropenol in 25 ml Diethylether und 5 ml Methanol werden unter Eisbadkühlung 25 ml der oben hergestellten Diazomethanlösung gegeben. Anschließend erfolgt die Zugabe von 53,4 mg (0,000139 mol) Bis(benzonitril)dichloro-palladium(II). Portionsweise werden anschließend weitere 28 ml der Diazomethanlösung zugesetzt. Nach etwa 1,5 Stunden wird das Lösemittel im Vakuum abdestilliert, der verbleibende Rückstand mit extrahiert, diese Lösung filtriert und das Lösemittel destillativ entfernt. Ausbeute: 4,25 g leicht gelbliche Kristalle **2a** (= 96% d. Th.)

### Beispiel 1: Benzilsäurecyclopropyltropinester-Methobromid:

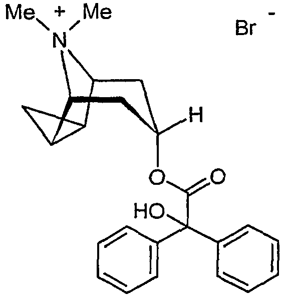

### 1.1.: Benzilsäuremethylester 3a:.

90 g (0,394 mol) Benzilsäure werden in 900 ml Acetonitril gelöst und bei 5°C tropfenweise mit 109,6 g (0,72 mol) DBU versetzt. Nach Zugabe von 204,4 g (1,44 mol) Methyljodid wird 24 Stunden bei Raumtemperatur (etwa 20-23°C) gerührt. Die Lösung wird bis zum Rückstand eingedampft, der Rückstand in Diethylether aufgenommen und mit Wasser extrahiert. Die organische Phase wird mit 5%iger wässeriger Natriumcarbonatlösung und Wasser gewaschen, getrocknet und das Lösemittel abdestilliert. Die Reinigung des Produkts erfolgt durch Umkristallisation aus Cyclohexan. Ausbeute: 77,19 g weiße Kristalle (= 81% d. Th.)
Schmp.: 74°-76° C.

### 1.2.: Benzilsäurecyclopropyltropinester 4a:

5,34 g (0,022 mol) Benzilsäuremethylester **3a**, 1,53 g (0,01 mol) **2a** und 0,25 g (0,01 mol) Natrium werden als Schmelze bei 75 mbar1 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit 10%-iger Kaliumhydrogensulfatlösung extrahiert, die daraus resultierende wässerige Phase basisch gestellt und mit Dichlormethan extrahiert. Die organische Phase wird abgetrennt, getrocknet und zur Trockene eingedampft. Die Reinigung des Produkts erfolgt durch Umkristallisation aus Acetonitril. Ausbeute: 2,41 g weiße Kristalle (= 66 % d. Th.).

### 1.3: Benzilsäurecyclopropyltropinester Methobromid :

0,46 g (0,0013 mol) **4a** werden in 5 ml Acetonitril aufgenommen und mit 1,53 g (0,0082 mol) 50%-iges Methylbromidlösung in Acetonitril in einem Druckreaktionsgefäß bei 80°C gerührt. Nach 2 Tagen wird die Lösung zur Trockene eingedampft, der Rückstand in Acetonitril aufgenommen und heiß filtriert. Nach Abkühlen werden die ausgefallenen Kristalle abgetrennt, getrocknet und aus Acetonitril umkristallisiert.

Ausbeute: 0,066 g weiße Kristalle (= 11 % d. Th); Schmp.: 208-209°C.

| | |
|---|---|
| Elementaranalyse: | berechnet: C (62,89) H (6,16) N (3,06) |
| | gefunden.: C (62,98) H (6,20) N (3,03). |

### Beispiel 2: 2,2-Diphenylpropionsäurecyclopropyltropinester -Methobromid:

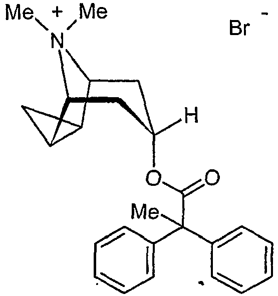

### 2.1.: 2,2-Diphenylpropionsäurechlorid 3b:.

Zu einer Suspension aus 25,0 g (0,11 mol) 2,2-Diphenylpropionsäure, 100 ml Dichlormethan und 4 Tropfen Dimethylformamid.werden bei 20° C 52,08g (0,33 mol) Oxalylchlorid langsam zugetropft. Es wird 1 h bei 20°C und 0,5 h bei 50° C gerührt. Das Lösungsmittel wird abdestilliert und der verbleibende Rückstand ohne weitergehende Reinigung in die nächste Stufe eingesetzt.

### 2.2: 2,2-Diphenylpropionsäurecyclopropyltropinester 4b:

2,3 g (0,015 mol) **2a** und 2,13 g (0,016 mol) Diisopropylethylamin werden in 30 ml Dichlormethan vorgelegt und innerhalb von 15 Minuten mit einer Lösung aus gemäß Schritt 2.1 hergestelltem Säurechlorid **3b** in Dichlormethan versetzt. Anschließend wird 2 Stunden bei Raumtemperatur und 72 Stunden bei 40° C gerührt. Zur Aufarbeitung wird mit Wasser gewaschen, über MgSO₄ getrocknet und das Lösemittel abdetilliert. Das Produkt wird mit einer Lösung von HCl in Diethylether in sein Hydrochlorid überführt. Zur Reinigung wird das gefällte Hydrochlorid in Wasser aufgenommen und mit Diethylether extrahiert. Die wässerige Phase wird mit 10%iger aq. Natriumcarbonatlösung basisch gestellt und mit Dichlormethan extrahiert. Die organische Phase wird über MgSO₄ getrocknet und das Lösemittel abdestilliert. Ausbeute: 2,15 g gelbes Öl (= 36% d. Th.)

### 2.3: 2,2-Diphenylpropionsäurecyclopropyltropinester Methobromid :

1,8 g (0,005 mol) der freien Base **4b** werden analog zur Durchführung unter Stufe 1.3 umgesetzt. Die Reinigung erfolgt durch Umkristallisation aus Acetonitril/Diethylether.

Ausbeute: 1,53 g weiße Kristalle (= 67 % d. Th.); Schmp.: 208-209°C;

| | |
|---|---|
| Elementaranalyse: | berechnet: C (65,79) H (6,63) N (3,07) |
| | gefunden.: C (65,47) H (6,77) N (3,03). |

### Beispiel 3: 9-Hydroxy-xanthen-9-carbonsäurecyclopropyltropinesterMethobromid :

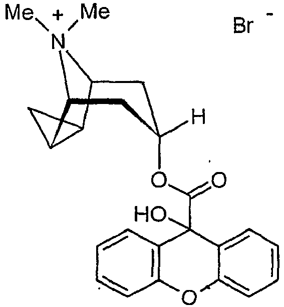

### 3.1.: 9-Hydroxy-xanthen-9-carbonsäuremethylester 3c:.

a) Xanthen-9-carbonsäuremethylester:
   Aus 21,75 g (0,95 mol) Natrium und 1500 ml Ethanol wird eine Natriumethylat-Lösung generiert. 214 g (0,95 mol) Xanthen-9-carbonsäure wird portionsweise in diese Lösung eingetragen und die erhaltene Suspension 1 Stunde bei Raumtemperatur nachgerührt. Danach wird der Feststoff abgetrennt, mit 1500 ml Diethylether gewaschen, die isolierten Kristalle in 1500 ml Dimethylformamid suspendiert und unter Rühren mit 126,73 ml (2,0 mol) Methyljodid versetzt. Die entstandene Lösung wird 24 Stunden bei Raumtemperatur stehengelassen, dann mit Wasser auf 6 l Gesamtvolumen verdünnt, kristallisiert, abgesaugt, mit Wasser gewaschen und getrocknet. Ausbeute: 167 g weiße Kristalle 7 (= 74% d. Th.) Schmp.: 82° C.
b) 9-Hydroxy-xanthen-9-carbonsäuremethylester **3c:**
   48,05 g (0,2 mol) Xanthen-9-carbonsäuremethylester werden in 1200 ml Tetrahydrofuran gelöst und bei 0° C mit 23,63 g (0,2 mol) Kalium-tert.butylat versetzt. Es wird anschließend 2 Stunden bei -10° bis -5° C Sauerstoff eingeleitet, danach mit 2 N wässeriger Salzsäure angesäuert und der größte Teil des Lösemittels destillativ entfernt. Der verbleibende Rückstand wird mit Ethylacetat und Wasser extrahiert, die organische Phase mit wässeriger Na₂S₂O₅-Lösung extrahiert, mit Wasser gewaschen, getrocknet und das Lösemittel abdestilliert. Die Reinigung des Produkts erfolgt durch Kristallisation aus Diisopropylether und Cyclohexan. Ausbeute: 11,10 g weiße Kristalle (= 22% d. Th.)

### 3.2: [9-Hydroxy-xanthen-9-carbonsäurecyclopropyltropinester 4c:

6,0 g (0,023 mol) **3c**, 3,065 g (0,02 mol) **2a** und 0,02 g Natrium werden in Analogie zu Stufe 1.2 zu **4c** umgesetzt. Ausbeute: 2,2 g weiße Kristalle(= 25 % d. Th.); Schmp.: 115-116°C.

### 3.3: 9-Hydroxy-xanthen-9-carbonsäurecyclopropyltropinester Methobromid :

2,1 g (0,006 mol) der freien Base **4c** werden analog zur Durchführung unter Stufe 1.3 umgesetzt. Die Reinigung erfolgt durch Umkristallisation aus Isopropanol. Ausbeute: 1,05 g beige Kristalle (= 37 % d. Th.); Schmp.: 218°C;

| | |
|---|---|
| Elementaranalyse: | berechnet: C (61,02) H (5,55) N (2,97) |
| | gefunden.: C (60,40) H (5,72) N (2,96). |

### Beispiel 4: 9-Methyl-fluoren-9-carbonsäurecyclopropyltropinester-Methobromid :

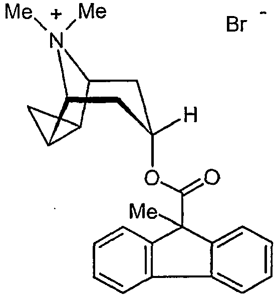

### 4.1.: 9-Methyl-fluoren-9-carbonsäure 3d:.

a) 9-Methyl-fluoren-9-carbonsäuremethylester:
   Aus 7,6 g (0,33 mol) Natrium und 300 ml Ethanol wird eine Natriumethylatlösung hergestellt, in die 69,6 g (0,33 mol) 9-Fluorencarbonsäure portionsweise zugegeben werden. Nach beendeter Zugabe wird 2,5 Stunden bei Raumtemperatur gerührt.
   Anschließend wird zur Trockene eingedampft, der Rückstand in 600 ml Dimethylformamid suspendiert und 93,96 g (0,662 mol) Methyljodid zugetropft. Es wird 3 Stunden bei konstanter Temperatur nachgerührt. Die trübe Lösung wird unter Kühlung in 500 ml Wasser und 300 ml Diethylether eingerührt und extrahiert, die organische Phase mit Wasser und 10%iger Natriumcarbonatlösung gewaschen,
   getrocknet und zur Trockene eingedampft. Der Rückstand wird mittels Säulenchromatographie, Laufmittel: Cyclohexan / Ethylacetat 96:4 gereinigt.
   Ausbeute: 12,61 g weiße Kristalle (= 16% d. Th.); Schmp.: 108°-109° C.
b) 9-Methyl-fluoren-9-carbonsäure **3d:**
   12,6 g (0,053 mol) 9-Methyl-fluoren-9-carbonsäuremethylester und 53 ml 2 molare, wässerige Natriumhydroxidlösung werden in 120 ml 1,4-Dioxan 24 Stunden bei Raumtemperatur gerührt. Das Dioxan wird abdestilliert, mit Wasser auf 300 ml Gesamtvolumen versetzt und mit Diethylether extrahiert. Die wässrige Phase wird mit 3 molarer, wässeriger HCl angesäuert, kristallisiert und filtriert.
      Ausbeute: 11,25 g weiße Kristalle (= 95% d. Th.); Schmp.: 168°-169° C.

### 4.2: : 9-Methyl-fluoren-9-carbonsäurecyclopropyltropinester 4d:

Aus 4,0 g (0,018 mol) **3d**, 4,53 g (0,036 mol) Oxalylchlorid und 4 Tropfen Dimethylformamid wird in 40 ml Dichlormethan das Säurechlorid hergestellt. 2,48 g (0,016 mol) **2a** und 1,91 g (0,019 mol) Triethylamin werden in 30 ml Dichlorethan suspendiert, das Säurechlorid in 30 ml Dichlorethan bei 30° C innerhalb von 15 Minuten zugetropft und anschließend 24 Stunden bei 40° C gerührt. Die Suspension wird mit Dichlormethan und Wasser extrahiert, die organische Phase mit essigsaurem Wasser gewaschen, getrocknet und das Lösemittel destillativ entfernt. Das Produkt wird in sein Hydrochlorid überführt: Zur Reinigung wird das gefällte Hydrochlorid in Wasser aufgenommen und mit Diethylether extrahiert. Die wässerige Phase wird basisch gestellt und mit Dichlormethan extrahiert. Die organische Phase wird über MgSO₄ getrocknet und das Lösemittel abdestilliert. Das Rohprodukt wird durch Umkristallisation aus Acetonitril gereinigt. Ausbeute: 1,81 g leicht beige Kristalle (= 30% d. Th.); Schmp.: 138°-139° C.

### 4.3: : 9-Methyl-fluoren-9-carbonsäurecyclopropyltropinester-Methobromid :

1,81 g (0,005 mol) der freien Base **4d** werden analog zur Durchführung unter Stufe 1.3 umgesetzt. Die Reinigung erfolgt durch Umkristallisation aus Acetonitril. Ausbeute: 1,26 g weiße Kristalle (= 56 % d. Th.); Schmp.: 228-229°C;

| | |
|---|---|
| Elementaranalyse: | berechnet: C (66,09) H (6,21) N (3,08) |
| | gefunden.: C (66,26) H (6,26) N (3,11). |

### Beispiel 5: 9-Methyl-xanthen-9-carbonsäurecyclopropyltropinester -Methobromid:

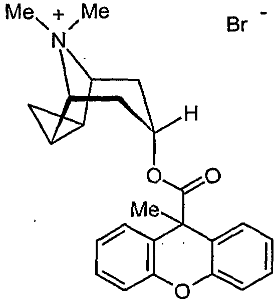

### 5.1.: 9-Methyl-xanthen-9-carbonsäure 3e:.

a) 9-Methyl-xanthen-9-carbonsäuremethylester:
   Ausgehend von 9,61 g (0,04 mol) 9-Xanthencarbonsäuremethylester (erhältlich nach Stufe 3.1.a) erfolgt die Umsetzung zur Titelverbindung in Analogie zur Vorgehensweise nach Stufe 4.1.a.
      Ausbeute: 6,05 g weiße Kristalle (= 60% d. Th.); Schmp.: 91-92°C.
b) 9-Methyl-xanthen-9-carbonsäure **3e:**
   Ausgehend von 20,34 g (0,08 mol) 9-Methyl-xanthen-9-carbonsäuremethylester erfolgt die Umsetzung zur Titelverbindung in Analogie zur Vorgehensweise nach Stufe 4.1.b. Ausbeute: 14,15 g weiße Kristalle (= 74% d. Th.); Schmp.: 207-208°C.

### 5.2 9-Methyl-xanthen-9-carbonsäurecyclopropyltropinester 4e:

Aus 5,0 g (0,021 mol) **3e**, 5,53 g (0,042 mol) Oxalylchlorid und 4 Tropfen Dimethylformamid wird in 50 ml Dichlormethan das Säurechlorid hergestellt. 3,06 g (0,02 mol) **2a** und das vorstehend generierte Säurechlorid werden in Analogie zur Vorgehensweise nach Stufe 4.2 zur Titelverbindung umgesetzt.
Ausbeute: 1,95 g leicht beige Kristalle(= 26 % d. Th.); Schmp.: 87-88°C.

### 5.3: 9-Methyl-xanthen-9-carbonsäurecyclopropyltropinester-Methobromid :

1,95 g (0,005 mol) der freien Base **4e** werden analog zur Durchführung unter Stufe 1.3 umgesetzt. Die Reinigung erfolgt durch Umkristallisation aus Acetonitril. Ausbeute: 0,54 g weiße Kristalle (= 23 % d. Th.); Schmp.: 193-194°C;

| | |
|---|---|
| Elementaranalyse: | berechnet: C (63,83) H (6,00) N (2,98) |
| | gefunden.: C (61,42) H (6,24) N (2,97). |

### Beispiel 6: 9-Hydroxy-fluoren-9-carbonsäurecyclopropyltropinester-Methobromid :

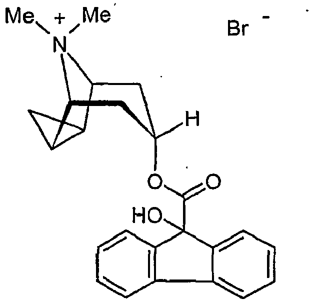

### 6.1.: 9-Hydroxy-fluoren-9-carbonsäuremethylester 3f:.

50,4 g (0,223 mol) 9-Hydroxy-9-fluorencarbonsäure werden in 500 ml Methanol gelöst, mit 5 ml (0,089 mol) konz. Schwefelsäure versetzt und 1 Stunde unter Rückfluß erhitzt. Nach Abkühlen werden 100 ml Natriumhydrogencarbonatlösung (ca. pH 8) zugegeben und das Methanol weitgehend eingedampft. Es wird mit Dichlormethan und Wasser extrahiert, die organische Phase getrocknet und zur Trockene eingedampft. Die Reinigung erfolgt durch Umkristallisation aus Ethylacetat.
Ausbeute: 50,0g weiße Kristalle (= 93% d. Th.).

### 6.2: : 9-Hydroxy-fluoren-9-carbonsäurecyclopropyltropinester 4f:

6,0 g (0,025 mol) **3f**, 3,45 g (0,023 mol) **2a** und 0,03 g Natrium werden in Analogie zu Stufe 1.2 zu **4f** umgesetzt. Die Reinigung erfolgt durch Umkristallisation aus Acetonitril. Ausbeute: 3,46 g weiße Kristalle(= 38 % d. Th.); Schmp.: 131-132°C.

### 6.3: 9-Hydroxy-fluoren-9-carbonsäurecyclopropyltropinester-Methobromid :

3,36 g (0,009 mol) der freien Base **4f** werden analog zur Durchführung unter Stufe 1.3 umgesetzt. Die Reinigung erfolgt durch Umkristallisation aus Isopropanol. Ausbeute: 3,32 g weiße Kristalle (= 79 % d. Th.); Schmp.: 219-220°C;

| | |
|---|---|
| Elementaranalyse: | berechnet: C (63,16) H (5,74) N (3,07) |
| | gefunden.: C (62,93) H (5,93) N (3,10). |

### Beispiel 7: 4,4'-Difluorbenzilsäuremethylestercyclopropyltropinester-Methobromid :

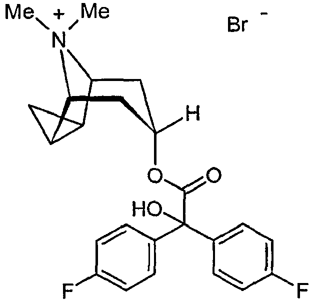

### 7.1.: 4,4'-Difluorbenzilsäuremethylester 3g:.

a) 4,4'-Difluorbenzilsäure:
   Zu einer Lösung aus 49,99 g (1,25 mol) NaOH-Plätzchen in 300 ml Wasser wird bei ca. 100° C eine Lösung aus 24,62 g (0,1 mol) 4,4'-Difluorbenzil in 250 ml Dioxan zugetropft und 2 h gerührt. Das Dioxan wird größtenteils abdestilliert und die verbleibende wässrige Lösung mit Dichlormethan extrahiert. Beim Ansäuern der wässrigen Lösung mit Schwefelsäure fällt ein Niederschlag aus, der abgesaugt, gewaschen und getrocknet wird. Das Filtrat wird mit Dichlormethan extrahiert, die organische Phase über Na₂SO₄ getrocknet und zur Trockene eingedampft.
      Ausbeute: 25,01 g (= 95 % d. Th.); Smp.: 133°-136° C
b) 4,4'-Difluorbenzilsäuremethylester **3g:**
   Zu frisch hergestellter Natriumethanolatlösung aus 2,17 g (0,095 mol) Natrium und 200 ml Ethanol werden bei 20° C 25,0 g (0,095 mol) 4,4'-Difluorbenzilsäure zugegeben und 3 h gerührt. Die Lösung wird zur Trockene eingedampft, der Rückstand in DMF gelöst, bei 20° C tropfenweise mit 22,57 g (0,16 mol) Methyljodid versetzt und 24 h gerührt. In die entstandene Suspension wurden unter Eiskühlung 300 ml Wasser getropft, mit Diethylether extrahiert, die organische Phase mit Wasser gewaschen, über Na₂SO₄ getrocknet und zur Trockene eingedampft. Ausbeute: 21,06 g (= 80 % d. Th.).

### 7.2: 4,4'-Difluorbenzilsäuremethylestercyclopropyltropinester 4g:

6,2 g (0,022 mol) **3g**, 3,37 g (0,022 mol) **2a** und 0,051 g Natrium werden in Analogie zu Stufe 1.2 zu **4g** umgesetzt. Die Reinigung erfolgt durch Umkristallisation aus Acetonitril. Ausbeute: 4,15 g weiße Kristalle(= 47 % d. Th.); Schmp.: 120-121 °C.

### 7.3: : 4,4'-Difluorbenzilsäuremethylestercyclopropyltropinester Methobromid :

2,0 g (0,005 mol) der freien Base **4g** werden analog zur Durchführung unter Stufe 1.3 umgesetzt. Die Reinigung erfolgt durch Umkristallisation aus Ethanol/Diethylether.
Ausbeute: 1,8 g weiße Kristalle (= 73 % d. Th.); Schmp.: 206-207°C;

| | |
|---|---|
| Elementaranalyse: | berechnet: C (58,31) H (5,30) N (2,83) |
| | gefunden.: C (58,15) H (5,42) N (2,84). |

Wie gefunden wurde, stellen die erfindungsgemäßen Verbindungen der Formel 1 Antagonisten des M3- Rezeptors (Muskarinischer Rezeptorsubtyp 3) dar. Die erfindungsgemäßen Verbindungen weisen bezüglich der Affinität zum M3-Rezeptor Ki-Werte von kleiner 10nM auf. Diese Werte wurde entsprechend der nachfolgend beschriebenen Vorgehensweise bestimmt.

### Chemikalien

3H-NMS wurde von der Firma Amersham, Braunschweig, mit einer spezifischen Radioaktivität von 3071 GBq/mmol (83 Ci/mmol) bezogen. Alle weiteren Reagentien wurden von Serva, Heidelberg und von Merck, Darmstadt erhalten.

### Zellmembranen:

Wir setzten Zellmembranen von CHO Zellen (Chinese hamster ovary) ein, die mit den entsprechenden Genen der humanen muskarinischen Rezeptorsubtypen hm1 bis hm5 transfiziert waren (BONNER). Die Zellmembranen des gewünschten Subtypes wurden aufgetaut, resuspendiert per Hand mit einem Glas-Homogenisator und mit HEPES-Puffer auf eine Endkonzentration von 20-30 mg Protein/ml verdünnt.

### Rezeptorbindungsstudien:

Der Bindungsassay wurde in einem Endvolumen von 1 ml ausgeführt und setzte sich zusammen aus 100 µl unmarkierter Substanz in verschiedenen Konzentrationen, 100 µl Radioligand (3H-N-Methylscopolamin 2 nmol/L (3H-NMS), 200 µl Membranpräparation und 600 µl HEPES Puffer (20 mmol/L HEPES, 10 mmol/L MgCl2, 100 mmol/L NaCl, mit 1 mol/L NaOH auf pH 7.4 eingestellt).

Die unspezifische Bindung ermittelten wir durch 10 µmol/L Atropin.

Die Inkubation von 45 min. erfolgte bei 37°C in 96-well Mikrotiterplatten (Beckman, Polystyrol, Nr. 267001) als Doppelbestimmung. Die Inkubation endete durch Filtration mittels Inotech Zellernter (Typ IH 110) über Whatman G-7 Filter. Die Filter wurden mit 3 ml eisgekühltem HEPES Puffer gewaschen und vor der Messung getrocknet.

### Bestimmung der Radioaktivität:

Die Radioaktivität der Filtermatten wurden simultan mittels zweidimensionalem, digitalem Autoradiograph (Berthold, Wildbad, Typ 3052) gemessen.

### Auswertung:

Die Ki-Werte berechneten wir mit impliziten Gleichungen, die direkt vom Massenwirkungsgesetz abgeleitet wurden, mit dem Modell für die 1 Rezeptor 2 Liganden Reaktion (SysFit - Software, SCHITTKOWSKI).

### Literatur:

BONNER TI, New subtypes of muscarinic acetylcholine receptors Trends Pharmacol. Sci. 10, Suppl.: 11-15 (1989); SCHITTKOWSKI K Parameter estimation in systems of nonlinear equations Numer Math. 68: 129-142 (1994).

Die erfindungsgemäßen Verbindungen der Formel 1 zeichnen sich durch vielfältige Anwendungsmöglichkeiten auf therapeutischem Gebiet aus.

Hervorzuheben sind erfindungsgemäß solche Anwendungsmöglichkeiten, für welche die erfindungsgemäßen Verbindungen der Formel 1 aufgrund ihrer pharmazeutischen Wirksamkeit als Anticholinergikum bevorzugt zur Anwendung gelangen können.

Dies sind beispielsweise die Therapie von Asthma oder COPD (chronic obstructive pulmonary disease = chronisch obstruktive Lungenerkrankung). Die Verbindungen der allgemeinen Formel **1** können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei

Menstruationsbeschwerden zum Einsatz gelangen.

Von den vorstehend beispielhaft aufgeführten Indikationsgebieten, kommt der Therapie von Asthma und COPD mittels der erfindungsgemäßen Verbindungen der Formel 1 eine besondere Bedeutung zu.

Die Verbindungen der allgemeinen Formel 1 können allein oder in Kombination mit anderen erfindungsgemäßen Wirkstoffen der Formel 1 zur Anwendung gelangen. Gegebenenfalls können die Verbindungen der allgemeinen Formel 1 auch in Kombination mit weiteren pharmakologisch aktiven Wirkstoffen eingesetzt werden. Es handelt sich hierbei insbesondere um Betamimetica, Antiallergika, PAF-Antagonisten, PDE IV-Inhibitoren, Leukotrien-Antagonisten, p38 Kinase-Inhibitoren, EGFR-Kinase-Hemmer und Corticosteroiden, sowie Wirkstoffkombinationen davon.

Als Beispiel für Betamimetika, die erfindungsgemäß mit den Verbindungen der Formel **1** als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze, ihrer Solvate und/oder ihrer Hydrate. Besonders bevorzugt gelangen als Betamimetika solche Wirkstoffe in Kombination mit den erfindungsgemäßen Verbindungen der Formel **1** zur Anwendung, die ausgewählt sind aus der Gruppe bestehend aus Fenoterol, Formoterol, Salmeterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.

Erfindungsgemäß bevorzugt sind die Säureadditionssalze der Betamimetika beispielsweise ausgewählt aus der Gruppe bestehend aus Hydrochlorid, Hydrobromid, Sulfat, Phosphat, Fumarat, Methansulfonat und Xinafoat. Besonders bevorzugt sind die Salze im Falle des Salmeterols ausgewählt aus Hydrochlorid, Sulfat und Xinafoat, von denen das Xinafoate besonders bevorzugt ist. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylline, Roflumilast, Ariflo, Bay-198004, CP-325,366, BY343, D-4396 (Sch-351591), V-11294A und AWD-12-281. Bevorzugte PDE-IV-Inhibitoren sind ausgewählt aus der Gruppe bestehend aus Enprofylline, Roflumilast, Ariflo und AWD-12-281, wobei AWD-12-281 als Kombinationspartner mit der erfindungsgemäßen Verbindung der Formel **1** besonders bevorzugt ist. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-IV-Inhibitoren gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Im Rahmen der vorliegenden Erfindung werden unter Dopamin-Agonisten, die gegebenenfalls in Kombination mit den Verbindungen der Formel 1 zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel 1 zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt 4-(2-Chlorphenyl)-9-methyl-2-[3(4-morpholinyl)-3-propanon-1-yl]-6H-thieno-[3,2-f] [1,2,4]triazolo[4,3-a][1,4]diazepin, 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin.

Als Beispiel für EGFR-Kinase-Hemmer, die mit den erfindungsgemäßen Verbindungen der Formel **1** als Kombination zum Einsatz kommen können, seien als besonders bevorzugt genannt 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluorphenyl)amino]-7-[4-((S)-6-methyl-2-oxo-morpholin-4-yl)-butyloxy]-6-[(vinylcarbonyl)amino]-chinazolin, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopropylmethoxy-chinazolin und 4-[(3-Chlor-4-fluorphenyl)amino]-6-[3-(morpholin-4-yl)-propyloxy]-7-methoxychinazolin. Eine Bezugnahme auf die vorstehend genannten EGFR-Kinase-Hemmer schließt im Rahmen der vorliegenden Erfindung eine Bezugnahme auf deren gegebenenfalls existierende pharmakologisch verträgliche Säureadditionssalze ein. Unter den physiologischbzw. pharmakologisch verträglichen Säureadditionssalzen, die von den EGFR-Kinase-Hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der EGFR-Kinase-Hemmer ausgewählt aus den Salzen der Essigsäure, Salzsäure, Bromwasserstoffsäure, Schwefelsäure, Phosphorsäure und Methansulfonsäure.

Als Beispiel für p38 Kinase-Hemmer, die mit den erfindungsgemäßen Verbindungen der Formel **1** als Kombination zum Einsatz kommen können, seien als besonders bevorzugt genannt 1-[5-*tert*-Butyl-2-*p*-tolyl-2H-pyrazol-3-yl]-3-[4-(2-morpholin-4-ylethoxy)naphthalin-1-yl]-harnstoff; 1-[5-*tert*-Butyl-2-*p*-tolyl-2H-pyrazol-3-yl]-3-[4-(2-(1-oxothiomorpholin-4-yl)ethoxy)naphthalin-1-yl]-harnstoff; 1-[5-*tert*-butyl-2-(2-methylpyridin-5-yl)-2H-pyrazol-3-yl]-3-[4-(2-pyridin-4-yl-ethoxy)naphthalin-1-yl]-harnstoff; 1-[5-*tert*-butyl-2-(2-methoxypyridin-5-yl)-2H-pyrazol-3-yl]-3-[4-(2-morpholin-4-yl-ethoxy)naphthalin-1-yl]-harnstoff oder 1-[5-*tert*-butyl-2-methyl-2H-pyrazol-3-yl]-3-[4-(2-morpholin-4-yl-ethoxy)naphthalen-1-yl]-harnstoff. Eine Bezugnahme auf die vorstehend genannten p38-Kinase-Hemmer schließt im Rahmen der vorliegenden Erfindung eine Bezugnahme auf deren gegebenenfalls existierende pharmakologisch verträgliche Säureadditionssalze ein. Unter den physiologisch bzw. pharmakologisch verträglichen Säureadditionssalzen, die von den p38-Kinase-hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind.

Werden die Verbindungen der Formel 1 in Kombination mit anderen Wirkstoffen eingesetzt, ist von den vorstehend genannten Verbindungsklassen die Kombination mit Steroiden, PDE IV-inhibitoren oder Betamimetika besonders bevorzugt. Der Kombination mit Betamimetika, insbesondere mit langwirksamen Betamimetika kommt dabei eine besondere Bedeutung zu. Als besonders bevorzugt ist die Kombination der erfindungsgemäßen Verbindungen der Formel 1 mit Salmeterol oder Formoterol anzusehen.

Geeignete Anwendungsformen zur Applikation der Verbindungen der Formel 1 sind beispielsweise Tabletten, Kapseln, Zäpfchen, Lösungen etc.
Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt. Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono-oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.
Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg /100 ml, bevorzugt unter 50 mg/100ml, besonders bevorzugt unter 20 mg/100ml. Generell sind solche Inhalationslösungen bevorzugt, in denen der Gehalt an Natriumedetat bei 0 bis 10mg/100ml liegt.
Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung:

### Pharmazeutische Formulierungsbeispiele

A)

| Tabletten | pro Tablette |
|---|---|
| Wirkstoff **1** | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.
B)

| Tabletten | pro Tablette |
|---|---|
| Wirkstoff **1** | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.
C)

| Ampullenlösung | |
|---|---|
| Wirkstoff **1** | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.
D)

| Dosieraerosol | |
|---|---|
| Wirkstoff **1** | 0,005 |
| Sorbitantrioleat | 0,1 |
| Monofluortrichlormethan und Difluordichlormethan 2:3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).
E)

| Lösungen (in mg/100ml) | |
|---|---|
| Wirkstoff **1** | 333.3 mg |
| Formoterolfumarat | 333.3 mg |
| Benzalkoniumchlorid | 10.0 mg |
| EDTA | 50.0 mg |
| HCl (1n) | ad pH 3.4 |

Diese Lösung kann in üblicher Art und Weise hergestellt werden.
F)

| Inhalationpulver | |
|---|---|
| Wirkstoff **1** | 6 µg |
| Formoterolfumarat | 6 µg |
| Lactose Monohydrat | ad 25 mg |

Die Herstellung des Inhaltionspulvers erfolgt in üblicher Art und Weise durch Mischen der einzelnen Bestandteile.
G)

| Inhalationpulver | |
|---|---|
| Wirkstoff **1** | 10 µg |
| Lactose Monohydrat | ad 5 mg |

Die Herstellung des Inhaltionspulvers erfolgt in üblicher Art und Weise durch Mischen der einzelnen Bestandteile.

## Patentansprüche

1. Verbindungen der allgemeinen Formel **1** 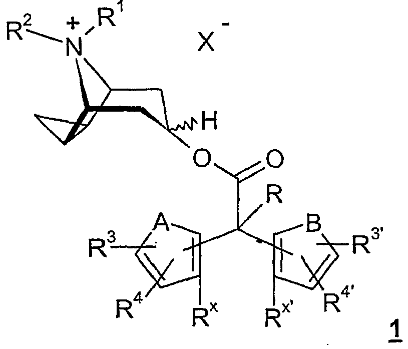 worin
X⁻ ein einfach negativ geladenes Anion,
A und B gleich oder verschieden, -O-, -S-, -NH-, -CH₂-, -CH=CH-, oder -N(C₁-C₄-Alkyl)-;
R Wasserstoff, Hydroxy, -C₁-C₄-Alkyl, -C₁-C₄-Alkyloxy, -C₁-C₄-Alkylen-Halogen, -O-C₁-C₄-Alkylen-Halogen, -C₁-C₄-Alkylen-OH, -CF₃, CHF₂, -C₁-C₄-Alkylen-C₁-C₄-alkyloxy, -O-COC₁-C₄-Alkyl, -O-COC₁-C₄-Alkylen-Halogen, -C₁-C₄-Alkylen-C₃-C₆-Cycloalkyl, -O-COCF₃ oder Halogen;
R¹ und R² gleich oder verschieden, -C₁-C₅-Alkyl, welches gegebenenfalls durch -C₃-C₆-Cycloalkyl, Hydroxy oder Halogen substituiert sein kann,
oder
R¹ und R² gemeinsam eine -C₃-C₅-Alkylen-Brücke;
R3, R4, R^{3'} und R^{4'}, gleich oder verschieden, Wasserstoff, C₁-C₄-Alkyl, C₁-C₄-Alkyloxy, Hydroxy, -CF₃, -CHF₂, CN, NO₂ oder Halogen;
R^{x} und R^{x'} gleich oder verschieden Wasserstoff, C₁-C₄-Alkyl, C₁-C₄-Alkyloxy, Hydroxy, -CF₃, -CHF₂, CN, NO₂ oder Halogen
oder
R^{x} und R^{x'} gemeinsam ein Einfachbindung oder eine verbrückende Gruppe ausgewählt aus den Brücken -O-, -S-, -NH-, -CH₂-, -CH₂-CH₂-, -N(C₁-C₄-Alkyl)-, -CH(C₁-C₄-Alkyl)- und -C(C₁-C₄-Alkyl)₂-, bedeuten.

2. Verbindungen der allgemeinen Formel **1** nach Anspruch 1, worin
X⁻ ein einfach negativ geladenes Anion ausgewählt aus der Gruppe Chlorid, Bromid, 4-Toluolsulfonat und Methansulfonat, bevorzugt Bromid;
A und B gleich oder verschieden, -O-, -S-, -NH- oder -CH=CH-;
R Wasserstoff, Hydroxy, -C₁-C₄-Alkyl, -C₁-C₄-Alkyloxy, -CF₃, -CHF₂, Fluor, Chlor oder Brom;
R¹ und R² gleich oder verschieden, C₁-C₄-Alkyl, welches gegebenenfalls durch Hydroxy, Fluor, Chlor oder Brom substituiert sein kann,
oder
R¹ und R² gemeinsam eine -C₃-C₄-Alkylen-Brücke;
R³, R⁴, R^{3'} und R^{4'}, gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom;
R^{x} und R^{x'} gleich oder verschieden Wasserstoff, C₁-C₄-Alkyl, C₁-C₄-Alkyloxy, Hydroxy, -CF₃, -CHF₂, CN, NO₂, Fluor, Chlor oder Brom
oder
R^{x} und R^{x'} gemeinsam ein Einfachbindung oder eine verbrückende Gruppe ausgewählt aus den Brücken -O-, -S-, -NH- und -CH₂-bedeuten.

3. Verbindungen der allgemeinen Formel **1** nach einem der Ansprüche 1 oder 2, worin
X⁻ ein einfach negativ geladenes Anion ausgewählt aus der Gruppe Chlorid, Bromid und Methansulfonat, bevorzugt Bromid;
A und B gleich oder verschieden, -S- oder -CH=CH-;
R Wasserstoff, Hydroxy, Methyl, Ethyl, Methyloxy, Ethyloxy, -CF₃, oder Fluor;
R¹ und R² gleich oder verschieden, Methyl, Ethyl, -CH₂F oder -CH₂-CH₂F, bevorzugt Methyl oder Ethyl;
R³, R⁴, R^{3'} und R^{4'}, gleich oder verschieden, Wasserstoff, Methyl, Methyloxy, -CF₃ oder Fluor;
R^{x} und R^{x'} gleich oder verschieden Wasserstoff, Methyl, Methyloxy, -CF₃ oder Fluor
oder
R^{x} und R^{x'} gemeinsam ein Einfachbindung oder die verbrückende Gruppe -O- bedeuten.

4. Verbindungen der allgemeinen Formel **1** nach einem der Ansprüche 1 bis 3, worin
X⁻ ein einfach negativ geladenes Anion ausgewählt aus der Gruppe Chlorid, Bromid und Methansulfonat, bevorzugt Bromid;
A und B gleich oder verschieden, -S- oder -CH=CH-;
R Wasserstoff, Hydroxy oder Methyl;
R¹ und R² gleich oder verschieden, Methyl oder Ethyl;
R3, R⁴, R^{3'} und R^{4'}, gleich oder verschieden, Wasserstoff, -CF₃ oder Fluor;
R^{x} und R^{x'} gleich oder verschieden Wasserstoff, -CF₃ oder Fluor;
oder
R^{x} und R^{x'} gemeinsam ein Einfachbindung oder die verbrückende Gruppe -O- bedeuten.

5. Verbindungen der allgemeinen Formel **1** nach einem der Ansprüche 1 bis 4, worin
X⁻ Bromid;
A und B -CH=CH-;
R Wasserstoff, Hydroxy oder Methyl;
R¹ und R² Methyl;
R³, R⁴, R^{3'} und R^{4'}, gleich oder verschieden. Wasserstoff oder Fluor;
R^{x} und R^{x'} gleich oder verschieden Wasserstoff oder Fluor oder
R^{x} und R^{x'} gemeinsam ein Einfachbindung oder die verbrückende Gruppe -O- bedeuten.

6. Verbindungen der allgemeinen Formel **1** nach einem der Ansprüche 1 bis 5, gegebenenfalls in Form der einzelnen optischen Isomeren, Mischungen der einzelnen Enantiomeren oder Racemate sowie gegebenenfalls in Form ihrer pharmakologisch verträglichen Säureadditionssalze.

7. Verbindung der allgemeinen Formel **1** gemäß einem der Ansprüche 1 bis 6 zur Verwendung als Arzneimittel.

8. Verwendung einer Verbindung der allgemeinen Formel **1** gemäß einem der Ansprüche 1 bis 6 zur Herstellung eines Arzneimittels zur Behandlung von Erkrankungen, in denen Anticholinergika einen therapeutischen Nutzen entfalten können.

9. Verwendung einer Verbindung der allgemeinen Formel **1** gemäß einem der Ansprüche 1 bis 6 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

10. Pharmazeutische Zubereitungen, enthaltend als Wirkstoff eine oder mehrere Verbindungen der allgemeinen Formel **1** gemäß einem der Ansprüche 1 bis 6 oder deren physiologisch verträgliche Salze gegebenenfalls in Kombination mit üblichen Hilfs- und/oder Trägerstoffen.

11. Pharmazeutische Zubereitungen nach Anspruch 10, **dadurch gekennzeichnet daß** diese neben einer oder mehrerer der Verbindungen der Formel **1** ferner wenigstens einen weiteren Wirkstoff enthalten, der ausgewählt ist aus der Gruppe der Betamimetica, Antiallergika, PAF-Antagonisten, PDE IV-Inhibitoren, Leukotrien-Antagonisten, p38 Kinase-Inhibitoren, EGFR-Kinase-Hemmern und Corticosteroiden.

12. Zwischenprodukte der allgemeinen Formel **4** 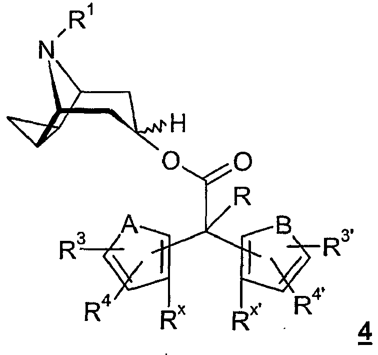 worin die Reste A, B, R, R¹, R³, R^{3'}, R⁴, R^{4'}, R^{x} und R^{x'} die in den Ansprüchen 1 bis 5 genannten Bedeutungen haben können., gegebenenfalls in Form ihrer Säureadditionssalze.

13. Verbindungen der allgemeinen Formel **2** 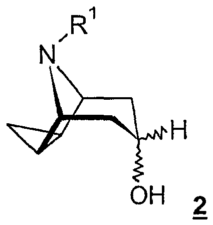 worin R¹ die in den Ansprüchen 1 bis 5 genannten Bedeutungen haben kann, gegebenenfalls in Form ihrer Säureadditionsalze.

## Claims

1. Compounds of general formula **1** 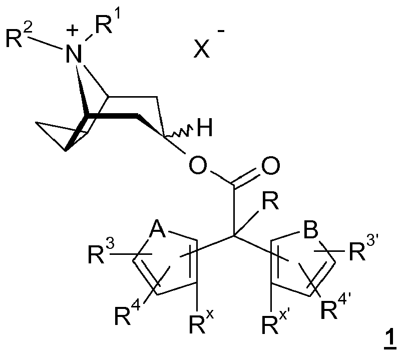 wherein
X⁻ denotes an anion with a single negative charge,
A and B which may be identical or different, denote -O, -S, -NH, -CH₂, -CH=CH, or -N(C₁-C₄-alkyl)-;
R denotes hydrogen, hydroxy, -C₁-C₄-alkyl, -C₁-C₄-alkyloxy, -C₁-C₄-alkylene-halogen, -O-C₁-C₄-alkylene-halogen, -C₁-C₄-alkylene-OH, -CF₃, CHF₂, -C₁-C₄-alkylene-C₁-C₄-alkyloxy, -O-COC₁-C₄-alkyl, -O-COC₁-C₄-alkylene-halogen, -C₁-C₄-alkylene-C₃-C₆-cycloalkyl, -O-COCF₃ or halogen;
R¹ and R² which may be identical or different, denote -C₁-C₅-alkyl, which may optionally be substituted by -C₃-C₆-cycloalkyl, hydroxy or halogen,
or
R¹ and R² together denote a -C₃-C₅-alkylene bridge;
R³, R⁴, R^{3'} and R^{4'}, which may be identical or different, denote hydrogen, C₁-C₄-alkyl, C₁-C₄-alkyloxy, hydroxy, -CF₃, -CHF₂, CN, NO₂ or halogen;
R^{x} and R^{x'} which may be identical or different, denote hydrogen, C₁-C₄-alkyl, C₁-C₄-alkyloxy, hydroxy, -CF₃, -CHF₂, CN, NO₂ or halogen
or
R^{x} and R^{x'} together denote a single bond or a bridging group selected from among the bridges -O, -S, -NH, -CH₂, -CH₂-CH₂-, -N(C₁-C₄-alkyl), -CH(C₁-C₄-alkyl)- and -C(C₁-C₄-alkyl)₂.

2. Compounds of general formula **1** according to claim 1, wherein
X⁻ denotes an anion with a single negative charge selected from among chloride, bromide, 4-toluenesulphonate and methanesulphonate, preferably bromide;
A and B which may be identical or different, denote -O, -S, -NH or -CH=CH-;
R denotes hydrogen, hydroxy, -C₁-C₄-alkyl, -C₁-C₄-alkyloxy, -CF₃, -CHF₂, fluorine, chlorine or bromine;
R¹ and R² which may be identical or different, denote C₁-C₄-alkyl, which may optionally be substituted by hydroxy, fluorine, chlorine or bromine,
or
R¹ and R² together denote a -C₃-C₄-alkylene-bridge;
R³, R⁴, R^{3'} and R^{4'}, which may be identical or different, denote hydrogen, C₁-C₄-alkyl, C1-C4-alkyloxy, hydroxy, -CF₃, -CHF₂, CN, NO₂, fluorine, chlorine or bromine;
R^{x} and R^{x'} which may be identical or different, denote hydrogen, C₁-C₄-alkyl, C₁-C₄-alkyloxy, hydroxy, -CF₃, -CHF₂, CN, NO₂, fluorine, chlorine or bromine
or
R^{x} and R^{x'} together denote a single bond or a bridging group selected from among the bridges -O, -S, -NH- and -CH₂- .

3. Compounds of general formula **1** according to one of claims 1 or 2,
wherein
X⁻ denotes an anion with a single negative charge selected from among chloride, bromide and methanesulphonate, preferably bromide;
A and B which may be identical or different, denote -S or -CH=CH-;
R denotes hydrogen, hydroxy, methyl, ethyl, methyloxy, ethyloxy, -CF₃, or fluorine;
R¹ and R² which may be identical or different, denote methyl, ethyl, -CH₂F or -CH₂-CH₂F, preferably methyl or ethyl;
R³, R⁴, R^{3'} and R^{4'}, which may be identical or different, denote hydrogen, methyl, methyloxy, -CF₃ or fluorine;
R^{x} and R^{x'} which may be identical or different, denote hydrogen, methyl, methyloxy, -CF₃ or fluorine
or
R^{x} and R^{x'} together denote a single bond or the bridging group -O-.

4. Compounds of general formula **1** according to one of claims 1 to 3, wherein
X⁻ denotes an anion with a single negative charge selected from among chloride, bromide and methanesulphonate, preferably bromide;
A and B which may be identical or different, denote -S or -CH=CH-;
R denotes hydrogen, hydroxy or methyl;
R¹ and R² which may be identical or different, denote methyl or ethyl;
R³, R⁴, R^{3'} and R^{4'}, which may be identical or different, denote hydrogen, -CF₃ or fluorine;
R^{x} and R^{x'} which may be identical or different, denote hydrogen, -CF₃ or fluorine; or
R^{x} and R^{x'} together denote a single bond or the bridging group -O.

5. Compounds of general formula **1** according to one of claims 1 to 4, wherein
X⁻ denotes bromide;
A and B denote -CH=CH-;
R denotes hydrogen, hydroxy or methyl;
R¹ and R² denote methyl;
R³, R⁴, R^{3'} and R^{4'}, which may be identical or different, denote hydrogen or fluorine;
R^{x} and R^{x'} which may be identical or different, denote hydrogen or fluorine
or
R^{x} and R^{x'} together denote a single bond or the bridging group -O.

6. Compounds of general formula **1** according to one of claims 1 to 5, optionally in the form of the individual optical isomers, mixtures of the individual enantiomers or racemates as well as optionally in the form of the pharmacologically acceptable acid addition salts thereof.

7. Compound of general formula **1** according to one of claims 1 to 6 for use as a medicament.

8. Use of a compound of general formula **1** according to one of claims 1 to 6 for preparing a medicament for the treatment of diseases in which anticholinergics can develop a therapeutic benefit.

9. Use of a compound of general formula **1** according to one of claims 1 to 6 for preparing a medicament for the treatment of asthma, COPD, vagally induced sinus bradycardia, heart rhythm disorders, spasms in the gastrointestinal tract, spasms in the urinary tract and menstrual pain.

10. Pharmaceutical preparations, containing as active substance one or more compounds of general formula **1** according to one of claims 1 to 6 or the physiologically acceptable salts thereof optionally in combination with conventional excipients and/or carriers.

11. Pharmaceutical preparations according to claim 10, **characterised in that** they contain, in addition to one or more of the compounds of formula **1**, at least one other active substance which is selected from among the betamimetics, antiallergics, PAF antagonists, PDE IV inhibitors, leukotriene antagonists, p38 kinase inhibitors, EGFR kinase inhibitors and corticosteroids.

12. Intermediate products of general formula **4** 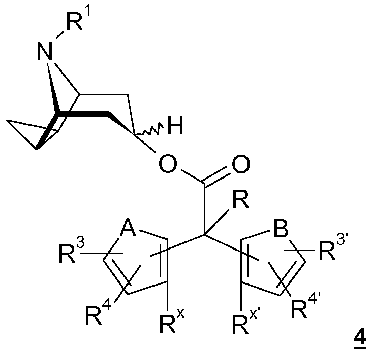 wherein the groups A, B, R, R¹, R³, R^{3'}, R⁴, R^{4'}, R^{x} and R^{x'} may have the meanings given in claims 1 to 5, optionally in the form of the acid addition salts thereof.

13. Compounds of general formula **2** 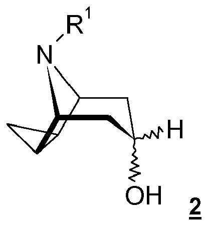 wherein R¹ may have the meanings given in claims 1 to 5, optionally in the form of the acid addition salts thereof.

## Revendications

1. Composés de formule générale 1 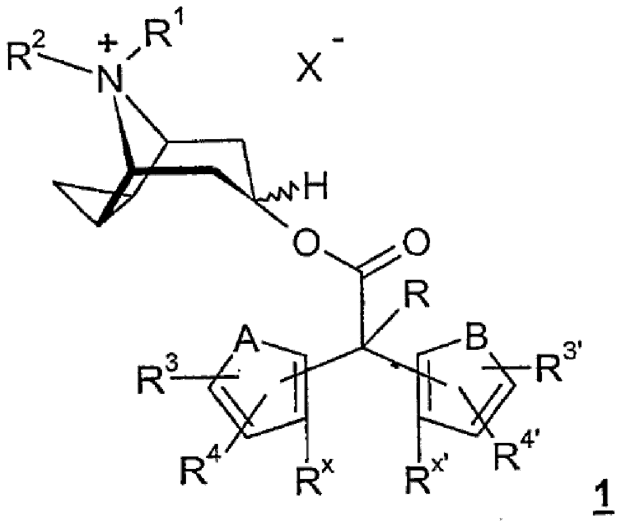 dans laquelle
X⁻ représente un anion à charge négative simple
A et B, identiques ou différents, représentent -0-, -S-, -NH-, -CH₂-, -CH=CH-, ou un groupe -N (alkyle en C₁ à C₄)- ;
R représente un atome d'hydrogène, un groupe hydroxy, alkyle en C₁ à C₄, alkyloxy en C₁ à C₄, alkylène en C₁ à C₄-halogéno, -O-alkylène en C₁ à C₄-halogéno, alkylène en C₁ à C₄-OH, -CF₃, CHF₂, alkylène en C₁ à C₄-alkyloxy en C₁ à C₄, -O-CO-alkyle en C₁ à C₄, -O-CO-alkylène en C₁ à C₄-halogéno, alkylène en C₁ à C₄-cycloalkyle en C₃ à C₆, -O-COCF₃ ou halogéno ;
R¹ et R², identiques ou différents, représentent un groupe alkyle en C₁ à C₅ qui peut être substitué éventuellement par un groupe cycloalkyle en C₃ à C₆, hydroxy ou halogéno, ou
R¹ et R² représentent conjointement un pont alkylène en C₃ à C₅ ;
R³, R⁴, R^{3'} et R^{4'}, identiques ou différents, représentent un atome d'hydrogène, un groupe alkyle en C₁ à C₄, alkyloxy en C₁ à C₄, hydroxy, - CF₃, -CHF₂, CN, NO₂ ou halogéno ;
R^{x} et R^{x'}, identiques ou différents, représentent un atome d'hydrogène, un groupe alkyle en C₁ à C₄, alkyloxy en C₁ à C₄, hydroxy, -CF₃, -CHF₂, CN, NO₂ ou halogéno ou
R^{x} et R^{x'} représentent conjointement une liaison simple ou un groupe ponté choisi parmi les ponts - 0-, -S-, -NH-, -CH₂-, -CH₂-CH₂-, -N (alkyle en C₁ à C₄) -, -CH (alkyle en C₁ à C₄) et -C (alkyle en C₁ à C₄)₂-.

2. Composés de formule générale 1 selon la revendication 1, dans laquelle
X⁻ représente un anion à charge négative simple choisi dans le groupe chlorure, bromure, 4-toluène-sulfonate et méthane-sulfonate, de préférence bromure ;
A et B, identiques ou différents, représentent -O-, -S-, -NH- ou -CH=CH- ;
R représente un atome d'hydrogène, un groupe hydroxy, alkyle en C₁ à C₄, alkyloxy en C₁ à C₄, - CF₃, -CHF₂, un atome de fluor, de chlore ou de brome ;
R¹ et R², identiques ou différents, représentent un groupe alkyle en C₁ à C₄ qui peut être substitué éventuellement par un groupe hydroxy, un atome de fluor, de chlore ou de brome,
ou
R¹ et R² représentent conjointement un pont alylène en C₃ à C₄ ;
R³, R⁴, R^{3'} et R^{4'}, identiques ou différents, représentent un atome d'hydrogène, un groupe alkyle en C₁ à C₄, alkyloxy en C₁ à C₄, hydroxy, - CF₃, -CHF₂, CN, NO₂, un atome de fluor, de chlore ou de brome,
R^{x} et R^{x'}, identiques ou différents, représentent un atome d'hydrogène, un groupe alkyle en C₁ à C₄, alkyloxy en C₁ à C₄, hydroxy, -CF₃; -CHF₂, CN, NO₂, un atome de fluor, de chlore ou de brome,
ou
R^{x} et R^{x'} représentent conjointement une liaison simple ou un groupe ponté choisi parmi les ponts - O-, -S-, -NH- et -CH₂-.

3. Composés de formule générale 1 selon l'une des revendications 1 ou 2, dans laquelle
X⁻ représente un anion à charge négative simple choisi dans le groupe chlorure, bromure et méthane-sulfonate, de préférence bromure ;
A et B, identiques ou différents, représentent -S- ou - CH=CH- ;
R représente un atome d'hydrogène, un groupe hydroxy, méthyle, éthyle, méthyloxy, éthyloxy, - CF₃ ou un atome de fluor ;
R¹ et R², identiques ou différents, représentent un groupe méthyle, éthyle, -CH₂F ou -CH₂-CH₂F, de préférence un groupe méthyle ou éthyle ;
R³, R⁴, R^{3'} et R^{4'}, identiques ou différents, représentent un atome d'hydrogène, un groupe méthyle, méthyloxy, -CF₃ ou un atome de fluor ;
R^{x} et R^{x'}, identiques ou différents, représentent un atome d'hydrogène, groupe méthyle, méthyloxy, -CF₃ ou un atome de fluor
ou
R^{x} et R^{x'} représentent conjointement une liaison simple ou le groupe -O- ponté.

4. Composés de formule générale 1, selon l'une des revendications 1 à 3, dans laquelle
X⁻ représente un anion à charge négative simple choisi dans le groupe chlorure, bromure et méthane-sulfonate, de préférence bromure ;
A et B, identiques ou différents, représentent -S- ou - CH=CH- ;
R représente un atome d'hydrogène, un groupe hydroxy ou méthyle ;
R¹ et R², identiques ou différents, représentent un groupe méthyle ou éthyle ;
R³, R⁴, R^{3'} et R^{4'}, identiques ou différents, représentent un atome d'hydrogène, -CF₃ ou un atome de fluor ;
R^{x} et R^{x'}, identiques ou différents, représentent -CF₃ ou un atome de fluor ; ou
R^{x} et R^{x'} représentent conjointement une liaison simple ou le groupe -O- ponté.

5. Composés de formule générale 1 selon l'une des revendications 1 à 4, dans laquelle
X⁻ représente un bromure ;
A et B représentent -CH=CH- ;
R représente un atome d'hydrogène, un groupe hydroxy ou méthyle ;
R¹ et R² représentent un groupe méthyle ;
R³, R⁴, R^{3'} et R^{4'}, identiques ou différents, représentent un atome d'hydrogène ou de fluor ;
R^{x} et R^{x'}, identiques ou différents, représentent un atome d'hydrogène ou de fluor,
ou
R^{x} et R^{x'} représentent conjointement une liaison simple ou le groupe -O- ponté.

6. Composés de formule générale 1 selon l'une des revendications 1 à 5, éventuellement sous la forme des isomères optiques individuels, de mélanges d'énantiomères individuels ou de racémates et éventuellement sous la forme de leurs sels d'addition acides pharmacologiquement acceptables.

7. Composé de formule générale 1 selon l'une des revendications 1 à 6, pour son utilisation comme médicament.

8. Utilisation d'un composé de formule générale 1 selon l'une des revendications 1 à 6, pour la production d'un médicament pour le traitement de maladies dans lesquelles des anticholinergiques peuvent présenter une utilité thérapeutique.

9. Utilisation d'un composé de formule générale 1 selon l'une des revendications 1 à 6, pour la production d'un médicament pour le traitement de l'asthme, de la BPCO, des bradycardies sinusales de type vagal, des troubles du rythme cardiaque, des spasmes des voies gastro-intestinales, des spasmes des voies urinaires et des troubles menstruels.

10. Préparations pharmaceutiques, contenant comme substance active, un ou plusieurs composés de formule générale 1 selon l'une des revendications 1 à 6 ou leurs sels physiologiquement acceptables, éventuellement en combinaison avec des adjuvants et/ou véhicules habituels.

11. Préparations pharmaceutiques selon la revendication 10, **caractérisées en ce que** celles-ci contiennent, en plus d'un ou plusieurs des composés de formule 1, en outre au moins une autre substance active qui et choisie dans le groupe des bêtamimétiques, anti-allergiques, antagonistes de PAF, inhibiteurs de PDE IV, antagonistes des leucotriènes, inhibiteurs de la p38 kinase, inhibiteurs de l'EGFR kinase et corticoïdes.

12. Produits intermédiaires de formule générale 4 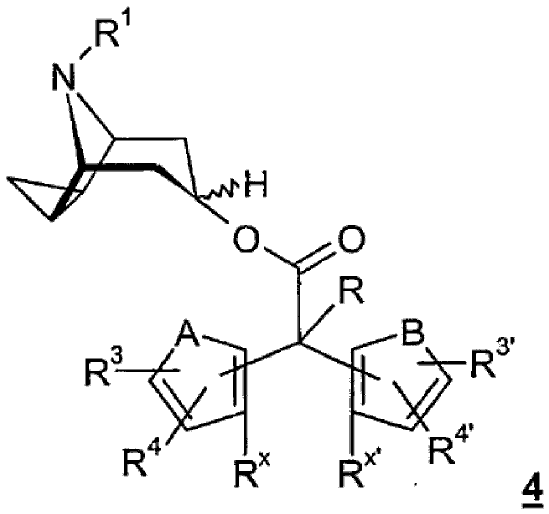 dans laquelle les radicaux A, B, R, R¹, R³, R^{3'}, R⁴, R^{4'}, R^{x} et R^{x'} peuvent avoir les significations indiquées dans les revendications 1 à 5, éventuellement sous la forme de leurs sels d'addition acide.

13. Composés de formule générale 2 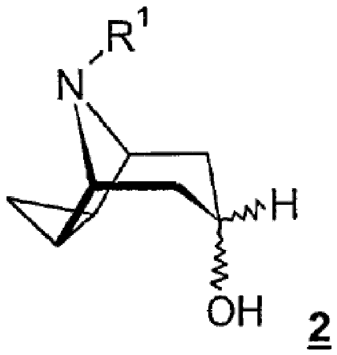 dans laquelle R¹ peut avoir les significations indiquées dans les revendications 1 à 5, éventuellement sous la forme de leurs sels d'addition acides.
